# EUROPEAN PATENT APPLICATION

(11) **EP 3 960 067 A1**
(43) Date of publication of application: **02.03.2022**
(21) Application number: 20193659.8
(22) Date of filing: 31.08.2020
(51) Int. Cl.: A61B 5/00, A61B 5/145

(54) **SWEAT SENSING DEVICE AND METHOD**

(71) Applicant: Koninklijke Philips N.V., 5656 AG Eindhoven (NL)
(72) Inventor: JOHNSON, Mark Thomas, 5656 AE Eindhoven (NL); VAN LIESHOUT, Ron Martinus Laurentius, 5656 AE Eindhoven (NL); PELSSERS, Eduard Gerard Marie, 5656 AE Eindhoven (NL); HUIJBREGTS, Laurentia Johanna, 5656 AE Eindhoven (NL); KROON, Bart, 5656 AE Eindhoven (NL)
(74) Representative: Philips Intellectual Property & Standards

(57) **Abstract**

Provided is a sweat sensing device (100). The device comprises a body (102) for positioning against skin (104). A recess (108) is defined in the body for receiving sweat from a skin area. The device further comprises an optical detection assembly (114) configured to detect a discrete sweat droplet (106) protruding into the recess from a sweat pore (110) in the skin area. Further provided is an optical sweat droplet sensing method.

## Description

### FIELD OF THE INVENTION

This invention relates to a sweat sensing device, and a sweat sensing method.

### BACKGROUND OF THE INVENTION

Non-invasive, semi-continuous and prolonged monitoring of biomarkers that indicate disease/health status and well-being is in demand for monitoring, for example, dehydration, stress, sleep, children's health and in perioperative monitoring.

Sweat, tear fluid and saliva may all be obtained non-invasively. Sweat is a particularly accessible biofluid, and is a rich source of information relating to the physiology and metabolism of a subject.

Some examples of clinical relevant components of sweat are Na⁺, Cl⁻ and/or K⁺ to monitor dehydration, lactate as an early warning for inflammation (which is relevant to sepsis), glucose for diabetics and neonates, and cortisol in relation to sleep apnoea and stress monitoring.

Continuous monitoring of high-risk patients, such as those with serious chronic conditions, pre- or post-operative patients, and the elderly, using sweat biomarker monitoring devices can provide higher quality diagnostic information than regular biomarker spot checks as normally done by repeatedly drawing multiple blood samples. Such continuous monitoring may be in a hospital setting or elsewhere. Human sweat alone or as mixture with sebum lipids may be an easily accessible source for biomarker measurements in wearable on-skin devices. For instance, cholesterol is an important biomarker associated with elevated risk in development of cardiovascular diseases. Inflammatory markers or cytokines, such as interleukins (e.g. TNF-a, IL-6) play an important role in the immune response and detection or disease monitoring of joint damage in rheumatoid and psoriatic arthritis, and bowel disease.

Examples of biomarkers that can be detected in eccrine/apocrine sweat using suitable capture species (antibodies, aptamers, molecular imprint polymers, etc.) are: small molecules such as urea, creatinine, cholesterol, triglycerides, steroid hormones (cortisol), glucose, melatonin; peptides and proteins, including cytokines such as IL-1 alpha, IL-1beta, IL-6, TNF alpha, IL-8 and TGF-beta IL-6, Cysteine proteinases, DNAse I, lysozyme, Zn-α2-glycoprotein, cysteine-rich secretory protein-3 and dermcidin; and large biomarkers such as the Hepatitis C virus.

As summarized by Mena-Bravo and de Castro in "Sweat: A sample with limited present applications and promising future in metabolomics", J. Pharm. Biomed. Anal. 90, 139-147 (2014), it has been found that the results from sweat sensing can be highly variable, and a correlation between values determined from blood and sweat samples appears to be lacking for various biomarkers. In this respect, historical studies in this area have involved relatively crude sampling techniques, such as collecting large sweat volumes in bags or textiles. Deficiencies in such techniques may have been a contributing factor to this apparent lack of correlation. The review of Mena-Bravo and de Castro thus highlights further key frustrations with conventional sweat sensing techniques in terms of the difficulty of producing enough sweat for analysis, the issue of sample evaporation, the lack of appropriate sampling devices, the need for trained staff, and issues relating to the normalization of the sampled volume.

Efforts have been made to address these issues by bringing wearable sensors into nearly immediate contact with sweat as it emerges from the skin. An example is the wearable patch presented by Gao et al. in "Fully integrated wearable sensor arrays for multiplexed in situ perspiration analysis", Nature 529, 509-514 (2016). The patch includes a sensor array for measuring Na⁺, K⁺, glucose, lactate, and skin temperature. However, the focus of this study is on the development and the integration of the sensors themselves which, whilst evidently crucial, does not address issues relating to sweat sample collection. The latter is mostly done by placing a several cm² sized absorbent pad between the skin and the sensor. The assumption is that, providing ample sweat is produced (hence tests are carried out on individuals that are exercising), the pad will absorb the sweat for analysis, and newly generated sweat will refill the pad and "rinse away" the old sweat. It is, however, likely that the time-dependent response of the sensor does not directly reflect the actual level of biomarkers over time because of accumulation effects. The sample collection and presentation to the published sensors may not be well-controlled so that continuous reliable sensing over a long period of time is difficult. Such patches may also not be designed to handle the tiny amounts of sweat that are produced under normal conditions, i.e. in the order of nanoliters per minute per sweat gland.

### SUMMARY OF THE INVENTION

It is an object of the invention to provide an improved sweat sensing device and method. The invention is defined by the independent claims. The dependent claims define advantageous embodiments.

According to an aspect there is provided a sweat sensing device comprising: a body for positioning against skin, a recess being defined in the body for receiving sweat from a skin area; and an optical detection assembly configured to detect a discrete sweat droplet protruding into the recess from a sweat pore in the skin area.

Since the optical detection assembly enables detection of a discrete sweat droplet protruding from an individual sweat pore, the sweat sensing device may enable monitoring of sweat production from an individual sweat gland from which the sweat pore extends.

Such optical interrogation of the sweat excretion behavior of an individual sweat gland may have various associated advantages, such as enabling determination of a sweat rate per sweat gland, and thereby, for instance, translating an analyte concentration in sweat to a calculated blood concentration. Alternatively or additionally, optical interrogation of the sweat excretion behavior of an individual sweat gland may be used in a process of identifying the sweat gland type, in other words eccrine or apocrine, of the sweat gland.

In an embodiment, the sweat sensing device comprises a fluidic system configured to transport the sweat droplet away from the recess following detection of the sweat droplet by the optical detection assembly. This may facilitate counting of sweat droplets successively produced in the skin area.

In a first set of examples, the optical detection assembly comprises: an optical detector; and a waveguide for carrying light to the optical detector, wherein the waveguide is arranged such that light is outcoupled therefrom upon contact between the waveguide and the sweat droplet protruding into the recess.

The outcoupling causes the light intensity detected by the optical detector to be decreased relative to the scenario in which no sweat droplet contacts the waveguide. This relatively straightforward detection principle benefits from being implementable using, for instance, a relatively simple and low cost optical detector, such as a photodiode.

The optical detection assembly may, for example, comprise a lighting unit for providing the light carried to the optical detector by the waveguide.

Alternatively or additionally, the sweat sensing device may comprise a window through which ambient light is provided into the waveguide. In this case, the ambient light may be carried to the optical detector by the waveguide. Use of ambient light in this manner may obviate the requirement for a powered lighting unit, thereby reducing the power consumption of the sweat sensing device.

The sweat sensing device may, in some embodiments, comprise a sweat collector and/or an analyte sensor configured to sense an analyte in sweat.

An outer surface of the waveguide, which partly delimits the recess, may be configured to transport the sweat droplet towards the sweat collector and/or the analyte sensor following detection of the sweat droplet by the optical detector.

Thus, the waveguide may serve a dual purpose: assisting with detection of the discrete sweat droplet, and transportation of the optically detected sweat droplet to a downstream sweat detector and/or analyte sensor.

The outer surface of the waveguide may comprise a surface tension gradient material for the transporting of the sweat droplet. Such a surface tension gradient material may, for example, be provided by the outer surface of the waveguide having hydrophilic and hydrophobic moieties thereon, which moieties are arranged to provide a wettability gradient along the outer surface.

The optical detection assembly may comprise a reference waveguide for carrying light to the optical detector. In this example, the reference waveguide is optically shielded from the recess such that the light carried by the reference waveguide is unaffected by sweat in the recess.

Such a reference waveguide may assist detection of the sweat droplet, since the intensity of the light passing though the reference waveguide provides a reference against which the light passing through the waveguide can be compared. This may facilitate identification of decreases in intensity of the light carried by the waveguide corresponding to discrete sweat droplets contacting the waveguide.

Such a reference waveguide may be particularly useful when ambient light is employed for the optical detection of the sweat droplet. This is because the reference waveguide enables the optical detection system to account for any changes in the ambient light level.

In another set of examples, the optical detection assembly comprises, as an alternative or in addition to the above-described waveguide/optical detector arrangement, an active pixel sensor array; and a lens arrangement configured to focus an image of the skin area received by the active pixel sensor array such as to enable detection of the sweat droplet.

An optical detection assembly which has imaging capability may benefit from capability to provide additional information to that provided by the waveguide/optical detector arrangement. For example, the active pixel sensor array may enable easier accessing of information which can be used to distinguish between sweat gland types.

The active pixel sensor array may, for example, be a charge-coupled device (CCD) sensor or a complementary metal oxide semiconductor (CMOS) sensor.

The sweat sensing device may comprise an optical window for transmitting light from the recess to the lens arrangement, wherein a surface of the optical window partly delimits the recess and is configured to transport the sweat droplet towards a sweat collector and/or an analyte sensor following detection of the sweat droplet by the optical detection assembly.

Thus, the optical window may serve a dual purpose: assisting with detection of the discrete sweat droplet, and transportation of the optically detected sweat droplet to a downstream sweat detector and/or analyte sensor.

The surface of the optical window may, for example, comprise a surface tension gradient material, e.g. of the type described above in relation to the first set of examples, for transporting the sweat droplet.

A lighting source may be included in the optical detection assembly for illuminating the recess. This may facilitate imaging of the sweat droplet.

In an embodiment, the sweat sensing device comprises: an analyte sensor for detecting the presence of an analyte in the sweat droplet which is specific to a single sweat gland type; and a processor configured to: identify, from data gathered by the optical detection assembly, a region of the skin area at which the sweat droplet is detected; and assign a sweat gland type to the region based on the detected presence of the analyte received from the analyte sensor.

Thus, the sweat gland type of the sweat gland responsible for excreting the sweat droplet can be assigned based on a sweat gland-specific analyte. Certain analytes are known to be excreted by one sweat gland type but not, or only in negligible quantities, by the other sweat gland type. For example, the sweat gland may be assigned to be either an eccrine or an apocrine gland based on detection, or otherwise, of such a sweat gland-specific analyte.

Alternatively or additionally, the sweat sensing device comprises: a processor configured to: determine, from data gathered by the optical detection assembly, a rate of growth of the sweat droplet; identify, from data gathered by the optical detection assembly, a region of the skin area at which the sweat droplet is detected; and assign a sweat gland type to the region based on the rate of growth of the sweat droplet.

It is known that under acute stress or pain conditions the eccrine glands are capable of producing sweat very quickly, with sweat being produced at multiple glands at large volume within around 2 seconds. If, for example, the optical detection assembly detects such a rapid and voluminous sweat production from a, or a group of, sweat glands within 1-2 seconds, all such glands may be labelled/assigned as eccrine glands.

Alternatively or additionally, the sweat sensing device comprises: a processor configured to: identify, from data gathered by the optical detection assembly, a region of the skin area at which the sweat droplet is detected; determine, from data gathered by the optical detection assembly, e.g., the above-described active pixel sensor array, a sweat gland type based on a sweat pore appearance characteristic detected in the region; and assign a sweat gland type to the region based on the sweat pore appearance characteristic.

In this case, the optical detection assembly is used to distinguish different optically detectable characteristics of the two sweat gland types in order to differentiate one from the other. The clearest differentiator is that the apocrine glands are attached to hair follicles and as such the majority of apocrine glands will produce a sweat droplet at a position where a hair is present. The sweat pore appearance characteristic may thus, for instance, comprise a parameter relating to a sweat pore hair follicle.

According to a further aspect there is provided a sweat sensing method comprising: arranging a body against skin such that a recess defined in the body receives sweat from a skin area; and optically detecting a discrete sweat droplet protruding into the recess from a sweat pore in the skin area.

### BRIEF DESCRIPTION OF THE DRAWINGS

Embodiments of the invention are described in more detail and by way of non-limiting examples with reference to the accompanying drawings, wherein:
FIG. 1 schematically depicts a cross-sectional view of a sweat sensing device according to an example;
FIG. 2 shows a graph of a signal from an optical detector included in the device shown in FIG. 1 versus time;
FIG. 3 schematically depicts a cross-sectional view of a sweat sensing device according to another example;
FIG. 4 schematically depicts a cross-sectional view of a sweat sensing device according to yet another example;
FIG. 5 shows a block diagram of a sweat sensing device according to a further example;
FIG. 6 provides a flowchart of a method according to an example;
FIG. 7 provides a flowchart of a method according to another example;
FIG. 8 provides a flowchart of a method according to yet another example; and
FIG. 9 provides a flowchart of a method according to a further example.

### DETAILED DESCRIPTION OF THE EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a sweat sensing device. The device comprises a body for positioning against skin. A recess is defined in the body for receiving sweat from a skin area. The device further comprises an optical detection assembly configured to detect a discrete sweat droplet protruding into the recess from a sweat pore in the skin area. Further provided is an optical sweat droplet sensing method.

Measuring analyte/biomarker concentration in the sweat can provide insights into medical and/or health conditions of a person. However, the concentration of certain biomarkers in blood is considered to give the best picture, in other words the "gold standard". The concentration in sweat may depend on the amount of sweat (higher sweat production leads to dilution), and on the source of sweat, for example whether the sweat originates from eccrine or apocrine glands. So in order to "calibrate" a sweat sensor, and to correctly perform the transformation/translation from a measured biomarker concentration in the sweat to a corresponding blood value, it may be necessary to estimate the rate of sweat production per sweat gland.

For this reason, it is proposed to incorporate an optical detection assembly capable of detecting a discrete sweat droplet protruding from a sweat pore into a sweat sensing device. In this manner, details of the sweat production at the level of a single sweat gland can be acquired. Such details may, for instance, be subsequently used for translation of biomarker concentrations to calculated blood values.

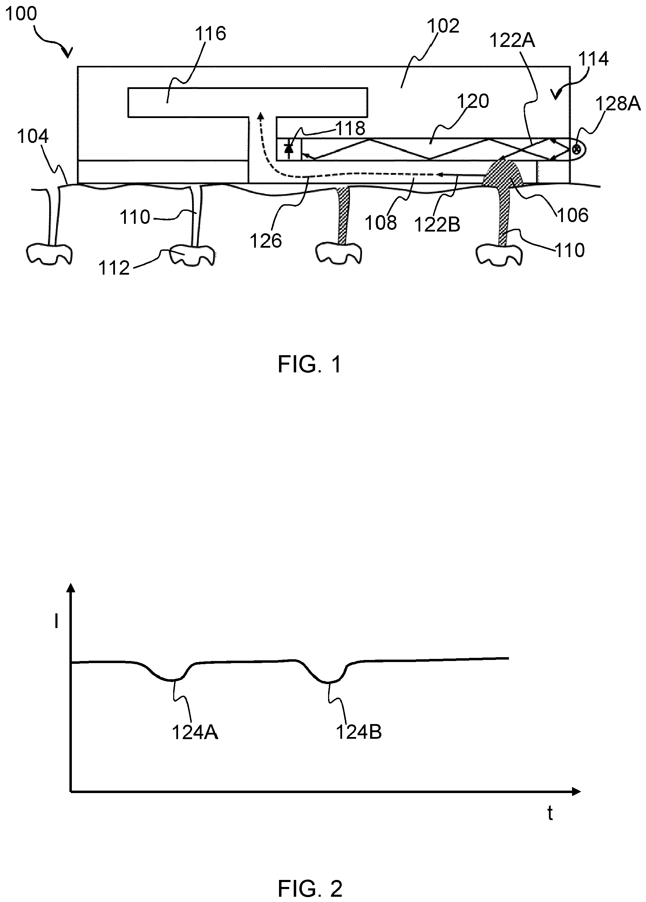

FIG. 1 shows a sweat sensing device 100 according to an example. The sweat sensing device 100 comprises a body 102. As shown in FIG. 1, the body 102 is positioned against the skin 104.

The body 102 may, for example, be included in a wearable patch which can be worn by a subject for sweat monitoring purposes. In such a non-limiting example, the sweat sensing device 100 may comprise, or in certain examples be defined by, the wearable patch.

In an embodiment, the body 102 is securable to the subject. For example, the body 102 may be adhered to the surface of the skin 104 using a suitable biocompatible adhesive. Alternatively, the body 102 may be held against the surface of the skin 104 by fastenings, e.g. straps, for attaching the body 102 to a body part of the subject.

The body 102 may be formed of any suitable material, e.g. a polymer, such as silicone rubber, capable of being disposed on the skin 104. In the case of a polymeric body 102, the body 102 may have sufficient flexibility so as to enable conformal application to the surface of the skin 104.

The material forming the body 102 and/or a coating on an exterior surface of the body 102 may be opaque to the wavelengths of light used to detect a sweat droplet 106. This is to minimise the possibility of extraneous ambient light interring with the optical detection of the sweat droplet 106. Optical detection of the sweat droplet 106 will be explained in more detail herein below.

A recess 108 is defined in the body 102. As shown in FIG. 1, the recess 108 provides a space in which a sweat droplet 106 can directly protrude from a sweat pore 110 from which the sweat droplet 106 is excreted. As schematically represented by the hashed area in FIG. 1, the sweat pore 110 carries sweat from a sweat gland 112 to the surface of the skin 104.

The sweat sensing device 100 comprises an optical detection assembly 114 configured to detect the discrete sweat droplet 106. The optical detection assembly 114 may have any suitable design provided that it is capable to detect a discrete, in other words individual, sweat droplet 106 protruding from a single sweat pore 110.

The detection principle of the optical detection assembly 114 may be non-imaging and/or imaging.

A non-imaging detection principle may benefit from simplicity and low cost. On the other hand, an optical detection assembly 114 which has imaging capability may benefit from capability to provide further information, e.g. information which may be used to more easily distinguish between sweat gland types, as will be explained in greater detail herein below.

The dimensions of the recess 108 are such that a discrete sweat droplet 106 protruding into the recess 108 is detectable by the optical detection assembly 114 without, or at least prior to, coalescing with one or more sweat droplets 106 protruding from neighboring sweat pore(s) 110.

Since the optical detection assembly 114 enables detection of a discrete sweat droplet 106 protruding from an individual sweat pore 110, the sweat sensing device 100 may enable monitoring of sweat production from an individual sweat gland 112.

The number of sweat droplets 106 can also be counted providing that the optically detected sweat droplets 106 drops are transported away from the detection region of the optical detection assembly 114 sufficiently quickly.

To this end, the sweat sensing device 100 comprises a fluidic system 116, e.g. a microfluidic system 116, configured to transport the optically detected sweat droplet 106 away from the recess 108.

In an embodiment, the fluidic system 116, e.g. microfluidic system, comprises an analyte sensor (not visible in FIG. 1) configured to sense an analyte in sweat. The analyte sensor may, for example, be configured to sense a concentration of an analyte contained in the sweat.

In a non-limiting example, the fluidic system 116 is configured to transport each sweat droplet 106 detected by the optical detection assembly 114 to the analyte sensor.

The optical detection assembly 114 may enable counting of the number of sweat droplets 106 being produced in the skin area, while the analyte sensor detects the concentration of the analyte in each sweat droplet 106 detected by the optical detection assembly 114. This sweat sensing device 100 may thus provide a single-device 100 solution for determining the analyte concentration per sweat gland 112.

In alternative examples, the analyte sensing may be performed by a separate sensor device.

The fluidic system 116 preferably transports each sweat droplet 106 at a rate which is faster that the rate of formation of a subsequent sweat droplet 106 from the same sweat pore 110.

More generally, the fluidic system 116 may, for instance, comprise a surface coated with a surface tension gradient material to assist transporting of the sweat droplet 106 away from the recess 108, e.g. towards the analyte sensor and/or a sweat collector (not visible in the Figures).

In particular, the surface along which the sweat droplet 106 is transported away from the recess 108 may have a chemical gradient down which the sweat droplet 106 migrates during use of the device 100.

An electrowetting technique may be used as an alternative or in addition to such a chemical gradient. Such an electrowetting technique uses an electric field to effect transient modification of the wetting properties of the surface in order to cause migration of the sweat droplet 106 away from the recess 108, and towards, for instance, the analyte sensor and/or sweat collector.

In an embodiment, the optical detection assembly 114 comprises an optical detector 118, and a waveguide 120 for carrying light to the optical detector 118. As shown in FIG. 1, the waveguide 120 is arranged such that light is outcoupled from the waveguide 120 upon contact being made between the sweat droplet 106 and an external surface of the waveguide 120.

In other words, the waveguide 120 is positioned on the skin-facing side of the device 100, and is spaced, due to the recess 108, slightly above the skin 104 at a position where one or more sweat glands 112 are present.

Any suitable optical detector 118 may be used for this purpose. The optical detector 118 may be configured to detect the intensity of the light being carried thereto by the waveguide 120. Accordingly, the optical detector 118 may comprise, or consist of, a photosensitive element, such as a photodiode.

This outcoupling is schematically depicted in FIG. 1. When light beam 122A is incident on a portion of the waveguide 120 contacted by the sweat droplet 106, the sweat droplet 106 causes outcoupling, such that the light beam 122B does not reach the optical detector 118. This is due to the refractive index of the waveguide 120 being sufficiently close to that of the sweat droplet 106 such that light carried in the waveguide 120 is refracted into the sweat droplet 106 when the sweat droplet 106 makes contact with the waveguide 120.

This refraction, as opposed to the total internal reflection which occurs at the waveguide 120-air interface in the absence of the sweat droplet 106, causes the light intensity detected by the optical detector 118 to be decreased relative to the scenario in which no sweat droplet 106 contacts the waveguide 120.

FIG. 2 shows a graph of a signal, e.g. an intensity signal I, from the optical detector 118 versus time t. Two separate dips in intensity 124A, 124B respectively correspond to two discrete sweat droplets 106 making contact with the waveguide 120. FIG. 2 thus demonstrates that the exemplary sweat sensing device 100 enables relatively straightforward counting of discrete sweat droplets 106 produced by a sweat gland or glands 112 in the skin area.

A processor (not visible in FIG. 1) included in the sweat sensing device 100 may, for example, be configured to count the number of sweat droplets 106 based on the number of fluctuations in the signal detected by the optical detector 118.

Moreover, the optical detection assembly 114 may enable estimation of the size or volume of the sweat droplet 106. Such an estimate may be made based on the magnitude of the reduction in intensity. The greater the reduction in intensity, the larger the sweat droplet 106.

Thus, in a non-limiting example, the processor may be configured to determine the volume of each detected sweat droplet 106 based on magnitude of change in the signal detected by the optical detector 118 corresponding to each discrete sweat droplet 106.

An outer surface of the waveguide 120 may, for example, be configured to transport the sweat droplet 106, following its detection by the optical detector 118, towards a sweat collector and/or analyte sensor included in the sweat sensing device 100. The direction of transportation of the sweat droplet 106 is represented in FIG. 1 by the hashed arrow 126.

The outer surface of the waveguide 120 may, for instance, comprise a surface tension gradient material for transporting the sweat droplet 106 towards the sweat collector and/or analyte sensor, as previously described.

Such a surface tension gradient material may, for example, be provided by the outer surface of the waveguide 120 having hydrophilic and hydrophobic moieties thereon, which moieties are arranged to provide a wettability gradient along the outer surface. For example, the outer surface may be functionalised with hydrophobic CH₃- moieties and hydrophilic OH-moieties in order to form a chemical gradient for transporting the sweat droplet 106 (Morgenthaler et al, Langmuir; 2003; 19(25) pp 10459-10462).

Such hydrophilic/hydrophobic domains may be provided at the molecular level, such that the wettability gradient varies substantially continuously along the outer surface of the waveguide 120. Such a chemical gradient may, for instance, be provided by grafted polymer chains functionalising the outer surface of the waveguide 120. Alternatively or additionally, hydrophilic/hydrophobic domains of µm dimensions may be provided on the outer surface such as to provide a stepwise wettability gradient. Preferably, the domains are arranged to have a gradual change in distribution over the length of the outer surface in the direction of the sweat collector and/or the analyte sensor.

In the case of the outer surface of the waveguide 120 comprising the surface tension gradient material, the waveguide 120, as well as being included in the optical detection assembly 114, forms a part of the fluidic system 116 of the sweat sensing device 100. This assists to improve the simplicity, and thus the manufacturability, of the sweat sensing device 100.

In an embodiment, the sweat sensing device 100 comprises a lighting unit 128A for providing light carried to the optical detector 118 by the waveguide 120. Any suitable lighting unit 128A may be used for this purpose, provided that the light emitted by the lighting unit 128A is detectable by the optical detector 118. For example, the lighting unit 128A may comprise a light emitting diode, filament bulb, etc.

In the non-limiting example shown in FIG. 1, the lighting unit 128A is positioned at a side of the body 102, and the waveguide 120 extends along at least part of a length of the recess 108. In other words, the waveguide 120 at least partly delimits the recess 108. The dimensions of the recess 108 may, for example, be such that at least one or two sweat pores 110 may excrete sweat thereinto.

Whilst not shown in FIG. 1, it is equally conceivable that light is supplied into the waveguide 120 by a plurality of lighting units 128A.

The number of sweat glands 112 per square centimetre varies per body location between 20 and 600, more typically 100 to 200.

The number of active sweat glands 112 depends on the body location, activity level and temperature. Typically, when the subject is in a sedentary state, about 10% of the sweat glands 112 are active.

Assuming sampling of the sweat from ten active sweat glands 112, then the required skin area can vary from 1 to 5 square centimetres to 0.2 to 0.5 square centimetres.

The length and width of the recess 108 may, for example, each be in the range of 2 to 100 mm, preferably 5 to 30 mm.

The area of the recess 108 may, for example, be 2 to 100 mm², preferably 5 to 30 mm².

In a non-limiting example, the skin area is circular, with a diameter in the range of 2 to 100 mm, preferably 5 to 30 mm.

The depth of the recess 108, across which the sweat droplet 106 protrudes from the sweat pore 110 towards the waveguide 120 may be, for instance, 10 to 100 µm, such as 20 to 30 µm.

It is conceivable, albeit considered unlikely, that two sweat droplets 106 from neighboring sweat glands 112 may contact the waveguide 120 simultaneously. In this case, the sweat sensing device 100 may only detect one of the sweat droplets 106. This risk may, for example, be reduced by controlling, i.e. reducing, the scale of the waveguide 120. For example, the sweat sensing device 100 may comprise a multiplicity of waveguide structures 120, as will be described in more detail herein below.

Thus, in the non-limiting example shown in FIG. 1, the optical detection assembly 114 may be regarded as a micro-optical component, which micro-optical component 114 takes the form of a waveguide 120 connected to an illumination source, in other words the lighting unit 128A, and an optical detector 118 comprising a photo-sensitive element, e.g. a photodiode.

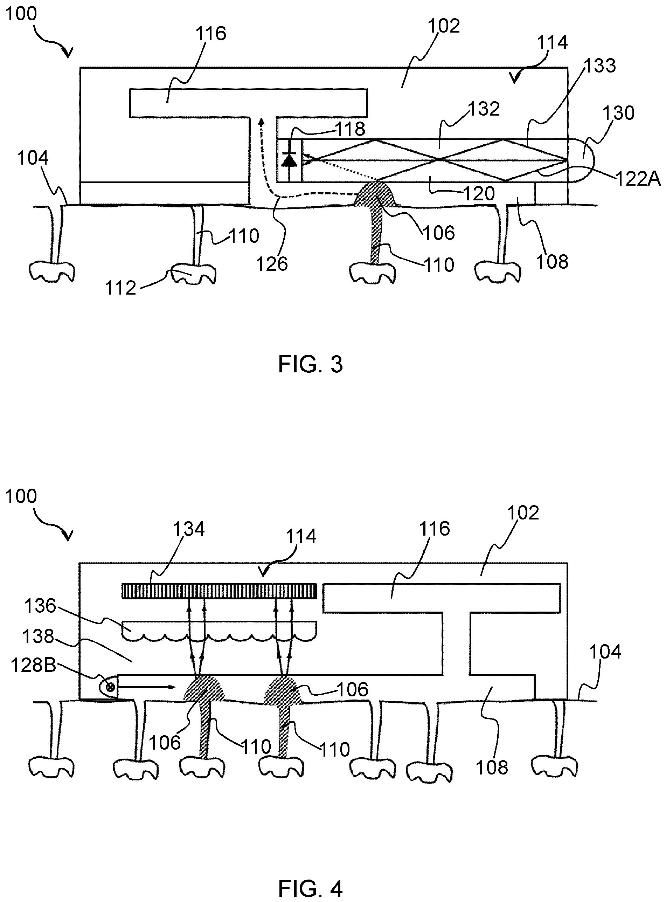

As an alternative or in addition to the lighting unit 128A shown in FIG. 1, the sweat sensing device 100 may include, as shown in FIG. 3, a window 130 through which ambient light is provided into the waveguide 120. In this case, ambient light is carried to the optical detector 118 by the waveguide 120.

Such a window 130 may obviate the requirement to include a powered lighting unit 128A of the type shown in FIG. 1. In this manner, the sweat sensing device 100 shown in FIG. 3 may benefit from improved, in other words lower, power consumption. This may assist to preserve the life of one or more batteries (not visible in the Figures) included in the device 100 for powering the onboard components of the device 100. This may be of particular importance when the sweat sensing device 100 is required to monitor a subject over relatively prolonged periods of time, such as over several hours or days.

Alternatively or additionally, the optical detection assembly 114 comprises a reference waveguide 132 for carrying light to the optical detector 118. The reference waveguide 132 may be optically shielded from the recess 108 such that the light carried by the reference waveguide 132 is unaffected by sweat in the recess 108.

In the non-limiting example shown in FIG. 3, the waveguide 120 and the reference waveguide 132 are shown extending parallel to each other across the recess 108, but it should be noted that the waveguide 120 and the reference waveguide 132 are optically separated from each other.

Whilst not visible in FIG. 3, the optical detector 118 may comprise a first optical sensing element, e.g. a photodiode, for sensing the light carried by the waveguide 120, and a second optical sensing element, e.g. a photodiode, for sensing the light carried by the reference waveguide 132.

Such a reference waveguide 132 may assist detection of the sweat droplet 106, since the intensity of the light passing though the reference waveguide 132 provides a reference against which the light passing through the waveguide 120 can be compared. This may facilitate identification of decreases in intensity of the light carried by the waveguide 120 corresponding to discrete sweat droplets 106 contacting the outer surface of the waveguide 120.

Such a reference waveguide 132 may be particularly useful in the non-limiting example shown in FIG. 3, in which ambient light is employed for the optical detection of the sweat droplet 106. This is because the reference waveguide 132 enables the optical detection system 114 to account for any changes in the ambient light level.

In other words, the reference light 133 carried by the reference waveguide 132 can be used as a calibration to account for any changes of ambient light level during the measurement.

More generally, when a processor is included in the sweat sensing device 100, the processor may be configured to detect the discrete sweat droplet 106 based on a comparison between the intensity of light carried by the reference waveguide 132 and the intensity of light carried by the waveguide 120.

As briefly mentioned above, the optical detection assembly 114 may have imaging capability to detect the discrete sweat droplet 106. In an embodiment, the optical detection assembly 114 comprises an active pixel sensor array 134 for imaging the skin area.

Any suitable active pixel sensor array 134 may be employed for this purpose. The active pixel sensor array 134 may, for example, comprise, or consist of, a charge-coupled device (CCD) sensor or a complementary metal oxide semiconductor (CMOS) sensor. Suitable active pixel sensor array 134 designs are known from, for instance, the field of digital cameras.

In the non-limiting example shown in FIG. 4, the optical detection assembly 114 comprises a lens arrangement 136 configured to focus an image of the skin area received by the active pixel sensor array 134 such as to assist detection of the sweat droplet 106. Any suitable lens arrangement 136 may be contemplated, such as an imaging micro-lens array.

The lens arrangement 136, e.g. micro-lens array, is positioned on the skin-facing side of the device 100, and is spaced, via the recess 108, slightly above the skin 104 at a position where sweat glands 112 are present and also reasonably in the focus of each lens of the lens arrangement 136. The lens arrangement 136 is correspondingly disposed between the active pixel sensor array 134 and the recess 108.

As shown in FIG. 4, the sweat sensing device 100 may also include a lighting source 128B for illuminating the recess 108. This may facilitate imaging of the skin area, and thus sweat droplet 106 detection. Any suitable lighting source 128B may be contemplated, such as a light emitting diode, filament bulb, etc.

Whilst the lighting source 128B is shown in FIG. 4 illuminating the recess 108 from one side, this is not intended to be limiting. The lighting source 128B may, for instance, be arranged to illuminate the recess 108 from two, e.g. opposing, sides.

An optical window 138 is provided between the lens arrangement 136 and the recess 108.

As represented by the arrows in FIG. 4, light which is reflected or scattered from the sweat droplet 106 passes through the lens arrangement 136, e.g. micro-lens array, which images the sweat droplet 106 onto the active pixel sensor array 134, e.g. CCD array.

The optical detection assembly 114 comprising, in the non-limiting example shown in FIG. 4, the active pixel sensor array 134, the lens arrangement 136, e.g. micro-lens array, and the optical window 138 may be regarded as an imaging micro-optical component which, when incorporated into the sweat sensing device 100, is capable of monitoring sweat production from individual sweat glands 112.

The size of the sweat droplet 106 can be measured by analysis of both the size of the image and the intensity of the light incident upon the active pixel sensor array 134. The number of sweat droplets 106 forming in the recess 108 and the on/off time of the sweat production from the individual sweat glands 112 can also be counted.

In general, the resolution of the active pixel sensor array 134, e.g. CCD array, which typically has a pitch of a few microns, may far exceed that of the lens arrangement 136, e.g. micro-lens array, which typically has a pitch of 10 to 100 µm. As a consequence, there may be many pixel elements capturing the light from a single micro-lens element. Computer optics approaches may therefore be used to further assess details of the sweat droplet 106 formation.

FIG. 4 may be regarded as illustrating a relatively simple imaging arrangement using non-polarized illumination, but this is not intended to be limiting. It is also possible for the sweat sensing device 100 to employ polarized illumination and/or polarized light detection to increase the quality of the image and the detected signals.

Furthermore, whilst lateral illumination along a length of the recess 108 is illustrated in FIG. 4, illumination and detection could be carried out from a direction normal to the surface of the skin 104 by making use of a beam splitter optical arrangement, e.g. a polarized beam splitter optical arrangement. Such beam splitter optical arrangements are known in the field of optical storage systems.

In a non-limiting example, the optical detection assembly 114 comprises a photoplethysmography (PPG) module. In this example, the PPG module is operably coupled to the lighting source 128B, and at least some of the light emitted from the lighting source 128B is incident on the skin 104. Light reflected from the skin 104 is received by the active pixel sensor array 134. A processor, e.g. included in the PPG module, is configured to detect heart beat based on the frequency domain of the light received by the active pixel sensor array 134. The processor also detects the presence of a sweat droplet 106 in the recess 108 based on a change in the intensity of light received by the active pixel sensor array 134.

It is desirable that the optically detected sweat droplets 106 correspond to those in which an analyte is sensed. Thus, the sweat sensing device 100 may comprise the analyte sensor described above.

Similarly to the non-limiting examples depicted in FIGs. 1 and 3, the sweat sensing device 100 shown in FIG. 4 comprises a fluidic system 116, e.g. a microfluidic system, for transporting the optically detected sweat droplet 106 away from the recess 108. The sweat droplet 106 may be transported, for instance, to a sweat collector and/or an analyte sensor, as previously described.

A surface of the optical window 138 may be treated with a material with a surface tension gradient configured to transport the optically detected sweat droplet 106 towards a sweat collector and/or an analyte sensor. The material has already been described above in relation to the outer surface of the waveguide 120 depicted in FIG. 1. The analyte sensor may, for example, by included in the fluidic system 116, e.g. microfluidic system, and the surface tension gradient may transport the sweat droplet 106 thereto for analyte sensing.

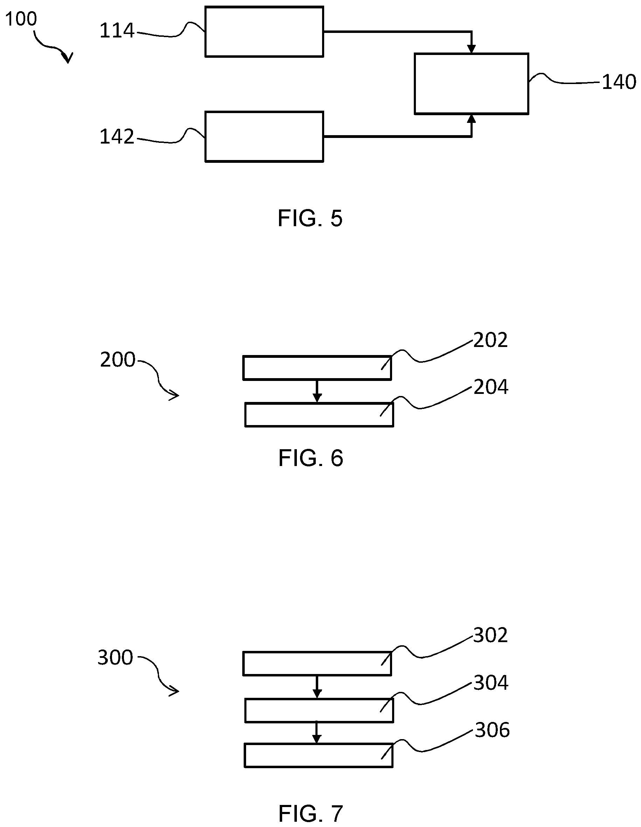

FIG. 5 shows a block diagram of a sweat sensing device 100. The sweat sensing device 100 comprises the above-described optical detection assembly 114, and a processor 140. Data from the optical detection assembly 114 is received by the processor 140, as represented in FIG. 5 by the arrow between the block 114 corresponding to the optical detection assembly and the block 140 corresponding to the processor.

The processor 140 may, for example, be configured to record the number of sweat droplets 106 and, in some examples, estimate the size or volume of each sweat droplet 106, as previously described.

In an embodiment, the processor 140 may be configured to determine, using the data received from the optical detection assembly 114, a sweat rate per sweat gland 112.

In the non-limiting example shown in FIG. 5, the sweat sensing device 100 comprises an analyte sensor 142. The analyte sensor 142 may be configured to sense an analyte in sweat.

The analyte sensor 142 may be included in the above-described fluidic system 116 housed within the body 102 of the sweat sensing devices 100 schematically depicted in FIGs. 1, 3, and 4.

The analyte sensor 142 is preferably configured to sense a concentration of an analyte contained in the sweat, as previously described.

More generally, data from the analyte sensor 142 is received by the processor 140, as represented in FIG. 5 by the arrow between the block 142 corresponding to the analyte sensor and the block 140 corresponding to the processor.

The processor 140 may, for example, be included in an onboard processing unit included in the body 102, e.g. when the body 102 is included in a wearable patch, and/or may be provided in an external data processing device which is physically separate from the body 102. In the latter case, the data from the optical detection assembly 114 and/or the analyte sensor 142 may be transmitted, e.g. wirelessly, to the external data processing device. Such wireless communication may, for instance, be via a suitable wireless communications protocol, e.g. Bluetooth^{®}.

It is desirable to determine whether the sweat is originating from eccrine or apocrine sweat glands 112. This is because these sweat gland types respectively produce sweat with different biomarker concentrations.

Both anatomical and functional differences have been found between the two types of sweat glands. The secretory coil of the eccrine sweat glands has three different cell types which all play a role in the secretion of sweat from eccrine sweat glands. One of these cell types, which can only be found in eccrine sweat glands, are the dark cells. The dark cells contain cytoplasmic electron-dense granules which are known for secreting many components such as glycoproteins, metals and the antimicrobial dermcidin. Dermcidin is an antimicrobial peptide secreted only by the eccrine sweat glands and directly attacks the bacteria on the skin. From the ten most abundant secreted proteins in our sweat, 45% is dermcidin. Dermcidin cannot be isolated from apocrine sweat and is not expressed in apocrine sweat glands Dermcidin is therefore a suitable analyte/biomarker for identifying eccrine sweat glands.

In addition, several proteins and peptides, e.g. cysteine proteinases, DNAse I, lysozyme, Zn-α2-glycoprotein, cysteine-rich secretory protein-3 and dermcidin have been identified in eccrine sweat.

Indications of clinically relevant unique apocrine sweat components are emerging, see Table 2 below. The apocrine gland secretes a translucent turbid viscous liquid onto the skin with a pH of 5 to 6.5 albeit in minute quantities. Unlike eccrine sweat glands, which secrete continuously, the apocrine glands secrete in periodic spurts. The apocrine sweat appears on the skin surface mixed with sebum, as sebaceous glands open into the same hair follicle. It is likely that the turbidity is caused by the non-dissolvable non-aqueous components, for instance the fatty acids of the sebum are not soluble in water. There is still a debate ongoing about the existence of a third gland type in the axilla; apoeccrine glands. In this case the analysis would be about determining the concentration of components in a mixture of apocrine and apoeccrine sweat, correcting for dilution by eccrine glands.

**Table 2: Examples of biomarkers present in apocrine sweat**

| Marker | Comments | Reference |
|---|---|---|
| Proteins | In general also present in eccrine sweat | J Invest Dermatol. 1953 Apr;20(4):285-97 |
| Apocrine secretion odor-binding proteins 1 and 2 (ASOB1 and ASOB2) | | Proc NatI Acad Sci USA. 1996 Jun 25;93(13):6626-30 |
| Carbohydrate | | J Invest Dermatol. 1953 Apr;20(4):285-97 |
| Ferric ions | | J Invest Dermatol. 1953 Apr;20(4):285-97 |
| Lipids | | Steroids34, 249-258 (1979) |
| Steroids | | Steroids34, 249-258 (1979) |
| Sialomucin | Constantine also showed presence in Eccrine sweat but in much lower concentration | Dermatology in General Medicine, 8e J Invest Dermatol. 1966 Jun;46(6):536-41 |
| Cathelicidin | | DERMATOPATHOLOGY, December 2011, |

Apocrine glands can be found on limited number of body locations for instance the axilla. Of interest would be to establish the concentration of sweat components as solely excreted by the apocrine glands in the apocrine sweat. However due to the presence of eccrine glands as well, the apocrine sweat is diluted by an unknown factor, thereby making the determination of above mentioned concentration ambiguous.

The sweat sensing device 100 may be used in such a way that it is possible not only to identify when a sweat droplet 106 is produced, but also whether it originates from an eccrine or an apocrine gland. In this manner, the measured biomarker concentration, e.g. measured using the above-described analyte sensor 142, can be appropriately transformed to correlate with, for instance, blood biomarker concentrations.

To this end, the following exemplary approaches may be employed. The approaches may start with a calibration phase, used to differentiate apocrine from eccrine sweat glands.

The body 102, e.g. included in a wearable patch, of the sweat sensing device 100 may thus be positioned on a location of the skin 104 in which both apocrine and eccrine glands are present, e.g. on an axilla.

Accordingly, in an embodiment the analyte sensor 142 is configured to detect the presence of an analyte in the optically detected sweat droplet 106 which is specific to a single sweat gland type, e.g. apocrine or eccrine. In this non limiting case, the sweat sensing device 100 includes the above-described active pixel sensor array 134, and the processor 140 is configured to identify, from data gathered by the active pixel sensor array 134, a region of the skin area at which the sweat droplet 106 is detected.

In alternative embodiments variants of the non-imaging approach of FIGs. 1 to 3, using for example a multiplicity of waveguide structures 120, may advantageously replace the active pixel sensor array 134. Such a multiplicity of waveguide structures 120 may render it possible to assign different sweat pores 110 to different waveguides 120. Thus, mapping of the skin area becomes possible (albeit at lower resolution in comparison with that typically provided by the active pixel sensor array 134).

The processor 140 is further configured to assign a sweat gland type to the region based on the presence of the analyte, or otherwise, received from the analyte sensor 142.

Since the sweat droplet 106 is detected protruding from the sweat pore 110 into the recess 108, the detected region of the sweat droplet 106 corresponds to that of the sweat pore 110 and associated sweat gland 112.

The active pixel sensor array 134 may identify the region of the skin area at which the sweat droplet 106 is detected using, for instance, the coordinates of the sweat droplet 106 in the x,y plane of the active pixel sensor array 134, e.g. CCD array.

The sweat sensing device 100 is configured in this embodiment to note that a sweat droplet 106 has been produced and also the location/region of the sweat droplet 106 in the skin area. The sweat droplet 106 is then transported to the analyte sensor 142, e.g. via the microfluidic system 116, and the analyte/biomarker concentration established. If, for instance, apocrine specific biomarkers are present, the sweat gland 112 is labelled as apocrine, and otherwise it is labelled as eccrine.

In another embodiment, which may be used as an alternative to or in combination with the above-described sweat gland identification principle, the processor 140 is configured to determine, from data gathered by the optical detection assembly 114, a rate of growth of the sweat droplet 106, and identify, from data gathered by the optical detection assembly 114, a region of the skin area at which the sweat droplet 106 is detected. The processor 140 then assigns a sweat gland type to the region based on the rate of growth of the sweat droplet 106.

In this case, the sweat sensing device 100 is configured to note not only that a sweat droplet 106 has been produced and also the location/region of the sweat droplet 106, which may, for instance, be recorded in the x,y plane of the active pixel sensor array 134, but also the rate at which the sweat droplet 106 has been produced.

It is known that under acute stress or pain conditions the eccrine glands are capable of producing sweat very quickly, with sweat being produced at multiple glands at large volume within around 2 seconds. If, for example, the active pixel sensor array 134 detects such a rapid and voluminous sweat production from a sweat gland 112, or a group of sweat glands 112, within 1-2 seconds, all such sweat glands 112 may be labelled/assigned as eccrine glands.

In a non-limiting example, the sweat sensing device 100 also includes a galvanic skin response (GSR) sensor. Spikes measured with such a GSR sensor may be used, e.g. by the processor 140, to confirm that such a sweat peak indicative of sweat production from eccrine glands has just occurred.

In another embodiment, which may be used as an alternative to or in combination with either or both of the above-described sweat gland identification principles, the processor 140 is configured to identify, from data gathered by the optical detection assembly 114, a region of the skin area at which the sweat droplet 106 is detected, and determine, from data gathered by the optical detection assembly 114, e.g. the active pixel sensor array 134, a sweat gland type based on a sweat pore appearance characteristic detected in the region. The processor 140 then assigns a sweat gland type to the region based on the sweat pore appearance characteristic.

In this case, the imaging optics are preferably used to distinguish different visible characteristics of the two sweat gland types in order to differentiate one from the other. The clearest differentiator is that the apocrine glands are attached to hair follicles and as such the majority of apocrine glands will produce a sweat droplet 106 at a position where a hair is present. As the hair is usually somewhat longer (at least 500-10000 microns) than the typical opening at the mouth of an eccrine gland (which has a diameter around 50 microns) sweat droplets 106 produced at hair follicles may be determined as being produced by apocrine glands.

Accordingly, in a non-limiting example the sweat pore characteristic comprises a parameter relating to a sweat pore hair follicle. The parameter may, for instance, comprise a ratio of a detected dimension of the sweat pore hair follicle to a detected diameter of the sweat pore.

Following assignment/labelling of the sweat gland(s) 112 in the skin area, or at least most of the sweat glands 112 in the skin area (in the calibration phase), the processor 140 may be configured to use this information to establish which type of sweat gland 112 has produced sweat during a measurement phase.

In a non-limiting example, the processor 140 may be configured to determine whether or not to collect and/or measure analyte concentration from a given optically detected sweat droplet 106 and depending upon the sweat gland type determined during the calibration phase to be producing the sweat droplet 106.

Such a determination may, for instance, be made based on the measurement which is desired at the moment that production of the sweat droplet(s) 106 takes place.

FIG. 6 provides a flowchart of a method 200 according to an example. The method 200 comprises arranging 202 a body against skin such that a recess defined in the body receives sweat from the skin area, and optically detecting 204 a discrete sweat droplet protruding into the recess from a sweat pore in the skin area.

This method 200 may be implemented using, for instance, the sweat sensing device 100 according to any of the embodiments described above. It is noted that embodiments described herein in relation to the disclosed devices 100 are applicable to the methods, and embodiments described herein in relation to the disclosed methods are applicable to the devices 100.

FIG. 7 provides a flowchart of a method 300 according to another example. The method 300 comprises identifying 302, from data gathered by an optical detection assembly arranged to detect a discrete sweat droplet protruding from a sweat pore in a skin area, a region of the skin area at which the sweat droplet is detected. Since the sweat droplet is detected protruding from the sweat pore, the detected region corresponds to that of the sweat pore and associated sweat gland, as previously described.

The method 300 further comprises receiving 304 an indication of the presence of an analyte in the sweat droplet which is specific to a single sweat gland type. The single sweat gland type may be apocrine or eccrine, as previously described. In step 306, a sweat gland type is assigned to the region based on the indication of the presence of the analyte.

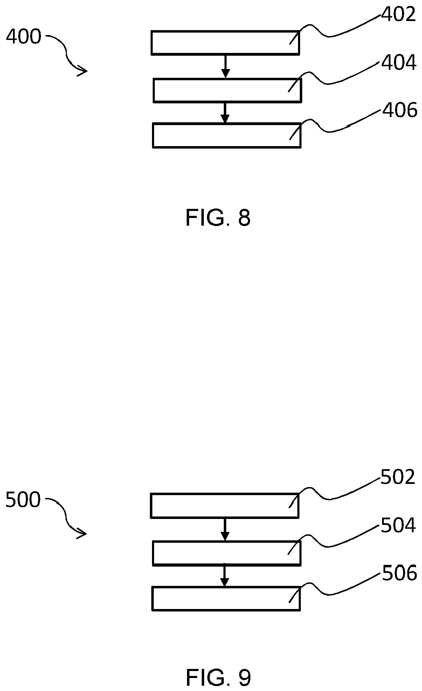

FIG. 8 provides a flowchart of a method 400 according to yet another example. The method 400 comprises identifying 402, from data gathered by an optical detection assembly arranged to detect a discrete sweat droplet protruding from a sweat pore in a skin area, a region of the skin area at which the sweat droplet is detected. The method 400 further comprises determining 404, from data gathered by the optical detection assembly, a rate of growth of the sweat droplet. In step 406, a sweat gland type is assigned to the region based on the rate of growth of the sweat droplet.

FIG. 9 provides a flowchart of a method 500 according to a further example. The method 500 comprises identifying 502, from data gathered by an optical detection assembly arranged to detect a discrete sweat droplet protruding from a sweat pore in a skin area, a region of the skin area at which the sweat droplet is detected. The method 500 further comprises determining 504, from data gathered by the optical detection assembly, e.g. an active pixel sensor array, a sweat gland type based on a sweat pore appearance characteristic detected in the region. In step 506, a sweat gland type is assigned to the region based on the sweat pore appearance characteristic.

The sweat pore appearance characteristic may, for example, comprise a parameter relating to a sweat pore hair follicle, as previously described.

Whilst not visible in the flowcharts, the method 300, 400, 500 may further comprise detecting an analyte concentration (during a subsequent measuring phase), and, using the assignment of the sweat gland type to the region, determining which type of sweat gland is responsible for producing the sweat having the detected analyte concentration.

Alternatively or additionally, the method 300, 400, 500 may comprise determining whether or not to collect and/or measure an analyte concentration from a given optically detected sweat droplet according to the sweat gland type determined (during the calibration phase) to be producing the sweat droplet. Such a determination may, for instance, be made based on the measurement which is desired at the moment that production of the sweat droplet(s) takes place.

One or more of the methods 300, 400, 500 may, for example, be implemented by the processor 140 included in the sweat sensing device 100 described above.

In summary, the invention proposes to incorporate a micro-optical component, e.g. including a micro-lens array 136 or waveguide 120 into a sweat sensing device 100. The micro-optical component is configured to pass optical information from a single sweat gland 112 to an optical sensor 118, 134. In this manner, details of the sweat production at single gland level (e.g. size of a sweat droplet 106 and/or differentiating details of an eccrine vs. an apocrine gland) can be accumulated, and optionally later used for translation of biomarker concentrations. The optical detection assembly/optical system 114 may be either imaging or non-imaging in nature, and may be configured to assess eccrine and/or apocrine glands, as previously explained.

The present disclosure may be applied for non-invasive, semi-continuous and prolonged monitoring of biomarkers that indicate health and well-being, for example for monitoring dehydration, stress, sleep, children's health and in perioperative monitoring. As well as being applicable for subject monitoring in general, the present apparatus, systems and methods may be specifically applied to provide an early warning for sudden deterioration of patients in the General Ward and Intensive Care Unit, or for investigation of sleep disorders. Currently, measurements may only be made in a spot-check fashion when a patient is visiting a doctor, although it is noted that the present disclosure may also be usefully applied in performing such spot-check measurements.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. Measures recited in mutually different dependent claims may advantageously be used in combination. Any reference signs in the claims should not be construed as limiting the scope.

## Claims

1. A sweat sensing device (100) comprising:
a body (102) for positioning against skin (104), a recess (108) being defined in the body for receiving sweat from a skin area; and
an optical detection assembly (114) configured to detect a discrete sweat droplet (106) protruding into the recess from a sweat pore (110) in the skin area.

2. The sweat sensing device (100) according to claim 1, comprising a fluidic system (116) configured to transport said sweat droplet (106) away from the recess (108) following detection of the sweat droplet by the optical detection assembly (114).

3. The sweat sensing device (100) according to claim 1 or claim 2, wherein the optical detection assembly (114) comprises:
an optical detector (118); and
a waveguide (120) for carrying light to the optical detector, wherein the waveguide is arranged such that light is outcoupled therefrom upon contact between the waveguide and said sweat droplet (106) protruding into the recess (108).

4. The sweat sensing device (100) according to claim 3, wherein the optical detection assembly (114) comprises a lighting unit (128A) for providing the light carried to the optical detector (118) by the waveguide (120).

5. The sweat sensing device (100) according to claim 3 or claim 4, comprising a window (130) through which ambient light is provided into the waveguide (120), said ambient light being carried to the optical detector (118) by the waveguide.

6. The sweat sensing device (100) according to any of claims 3 to 5, wherein an outer surface of the waveguide (120) partly delimits the recess (108), the outer surface being configured to transport said sweat droplet (106) towards a sweat collector and/or an analyte sensor following detection of the sweat droplet by the optical detector (118); optionally wherein said outer surface comprises a surface tension gradient material for said transporting of the sweat droplet.

7. The sweat sensing device (100) according to any of claims 3 to 6, comprising a reference waveguide (132) for carrying light to the optical detector (118), wherein the reference waveguide is optically shielded from the recess (108) such that the light carried by the reference waveguide is unaffected by sweat in the recess.

8. The sweat sensing device (100) according to any of claims 1 to 7, wherein the optical detection assembly (114) comprises:
an active pixel sensor array (134); and
a lens arrangement (136) configured to focus an image of the skin area received by the active pixel sensor array such as to enable detection of the sweat droplet (106).

9. The sweat sensing device (100) according to claim 8, comprising an optical window (138) through which light passes from the recess (108) to the lens arrangement (136), wherein a surface of the optical window partly delimits the recess, and is configured to transport said sweat droplet (106) towards a sweat collector and/or an analyte sensor following detection of the sweat droplet by the optical detection assembly (114); optionally wherein said surface comprises a surface tension gradient material for said transporting of the sweat droplet.

10. The sweat sensing device (100) according to claim 8 or claim 9, wherein the optical detection assembly (114) comprises a lighting source (128B) for illuminating the recess (108).

11. The sweat sensing device (100) according to any of claims 1 to 10, comprising:
an analyte sensor (142) for detecting the presence of an analyte in said sweat droplet (106) which is specific to a single sweat gland type; and
a processor (140) configured to:
identify, from data gathered by the optical detection assembly (114), a region of said skin area at which the sweat droplet is detected; and
assign a sweat gland type to said region based on said detected presence of the analyte received from the analyte sensor.

12. The sweat sensing device (100) according to any of claims 1 to 10, comprising:
a processor (140) configured to:
determine, from data gathered by the optical detection assembly (114), a rate of growth of said sweat droplet (106);
identify, from data gathered by the optical detection assembly, a region of said skin area at which the sweat droplet is detected; and
assign a sweat gland type to said region based on said rate of growth of the sweat droplet.

13. The sweat sensing device (100) according to any of claims 1 to 10, comprising:
a processor (140) configured to:
identify, from data gathered by the optical detection assembly (114), a region of said skin area at which the sweat droplet (106) is detected;
determine, from data gathered by the optical detection assembly, a sweat gland type based on a sweat pore appearance characteristic detected in the region; and
assign a sweat gland type to said region based on said sweat pore appearance characteristic.

14. The sweat sensing device according to claim 13, wherein the sweat pore appearance characteristic comprises a parameter relating to a sweat pore hair follicle.

15. A sweat sensing method (200) comprising:
arranging (202) a body against skin such that a recess defined in the body receives sweat from a skin area; and
optically detecting (204) a discrete sweat droplet protruding into the recess from a sweat pore in the skin area.
